# EUROPEAN PATENT APPLICATION

(11) **EP 3 677 203 A1**
(43) Date of publication of application: **08.07.2020**
(21) Application number: 20150024.6
(22) Date of filing: 02.01.2020
(51) Int. Cl.: A61B 17/34

(54) **SEALS FOR SURGICAL ACCESS DEVICES**

(30) Priority: 03.01.2019 US 201916238823
(71) Applicant: Covidien LP, Mansfield, MA 02048 (US)
(72) Inventor: EBERSOLE, Garrett, Hamden, CT 06417 (US); LaPierre, Nicolette, Windsor Locks, CT 06096 (US); DININO, Matthew A., Newington, CT 06111 (US); DESJARDIN, Kevin, Cheshire, CT 06410 (US); PILLETERE, Roy J., North Haven, CT 06473 (US); BROWN, Eric, Haddam, CT 06438 (US)
(74) Representative: Maschio, Antonio

(57) **Abstract**

A cannula includes an instrument valve housing and a valve assembly disposed within the instrument valve housing. The valve assembly includes a seal assembly (270) having at least one seal member (272,274,276) including a ring portion (272a,274a,276a) and a septum seal (272b,274b,276b) extending across the ring portion. The valve assembly further includes a guard assembly (130) and a centering mechanism. The guard assembly (130) includes a plurality of guard members. Each guard member includes a ring portion, and a flap portion having first and second flap sections and a groove disposed between the first and second flap sections. The centering mechanism includes an annular ring and a plurality of spokes extending outwardly from the annular ring. The assemblies are supported within the annular ring which is disposed within the instrument valve housing. The flap portions of the guard members are configured to engage and stretch the septum seal (270) during reception of a surgical instrument through the valve assembly.

## Description

### BACKGROUND

### Technical Field

The present disclosure relates to seals. More particularly, the present disclosure relates to seals for surgical access devices.

### Background

In order to facilitate minimally invasive surgery, a working space must be created in the desired surgical plane. An injection of an insufflation fluid, typically CO₂, can be used to create a pneumoperitoneum in the abdomen. To work in the created space, access ports are required for surgical instrumentation and cameras. These ports maintain the pressure creating the pneumoperitoneum with seals that adapt to the surgical instrumentation.

The breadth of surgical instrumentation on the market today requires a robust seal capable adjusting to multiple sizes and withstanding multiple insertions of surgical instrumentation. In addition, traditional access ports are susceptible to seal tears and pullouts, especially when used with clip appliers.

Therefore, it would be beneficial to have an access assembly with improved seal durability, increased instrument compatibility, robotic compatibility, obturator compatibility, minimized impact on manufacturing, future compatibility, and/or cannula connection compatibility, while maintaining the cost.

### SUMMARY

An improved access assembly is provided. The access assembly includes a cannula having an instrument valve housing and a valve assembly disposed within the instrument valve housing. More particularly, the valve housing includes first and second housing sections and defining a cavity, and the valve assembly is disposed within the cavity of the instrument valve housing. The valve assembly includes a seal assembly having at least one seal member. The at least one seal member includes a ring portion and a septum seal extending across the ring portion. The valve assembly also includes a guard assembly supporting the seal assembly. The guard assembly includes a plurality of guard members. Each guard member of the plurality of guard members has a ring portion and a flap portion. The flap portion of each guard member of the plurality of guard members includes first and second flap sections and a groove disposed between the first and second flap sections. The valve assembly further includes a centering mechanism having an annular ring and a plurality of spokes extending outwardly from the annular ring. The seal and guard assemblies are supported within the annular ring and the annular ring is disposed within the cavity of the instrument valve housing. The flap portions of the plurality of guard members of the guard assembly are configured to engage and stretch the septum seal during reception of a surgical instrument through the valve assembly.

In embodiments, the guard assembly includes first, second, third and fourth guard members. The ring portions of the first, second, third and fourth guard members may define an annular member. The first and second flap sections of the first, second, third and fourth guard members may be tapered radially inward. The groove in each of the first, second, third and fourth guard members may extend radially inward.

In some embodiments, at least one seal member of the seal assembly includes three seal members. Each of the three seal members may include a septum seal defining an opening having a substantial tear-drop shape. Each seal member of the three seal members may be disposed relative to the other seal members such that the openings in the septum seals are one-hundred twenty degrees apart.

The seal assembly of the valve assembly may include four seal members. Each of the four seal members includes a septum seal defining a sector of the septum seal. The sector of the septum seal may include one quarter of an area of the septum seal. Each seal member of the four seal members may be disposed relative to the other seal members such that the openings in the septum seals are oriented ninety degrees apart.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiments given below, serve to explain the principles of the disclosure, wherein:
FIG. 1 is a perspective side view of a cannula of a trocar assembly according to an embodiment of the present disclosure;
FIG. 2 is a perspective side view of an instrument valve housing and cannula assembly of the cannula shown in FIG. 1, separated;
FIG. 3 is a perspective side view of the instrument valve housing shown in FIG. 2, including first and second housing sections and a valve assembly;
FIG. 4 is a perspective side view of the valve assembly shown in FIG. 3;
FIG. 5 is a perspective side view of a guard assembly, a centering mechanism, and a seal assembly of the valve assembly shown in FIG. 3;
FIG. 6 is a perspective side view of the seal assembly and guard assembly shown in FIG. 5;
FIG. 7 is an exploded view of the guard assembly shown in FIGS. 5 and 6;
FIG. 8 is a perspective side view of a seal assembly according to another embodiment of the present disclosure, secured to the guard assembly shown in FIG. 5;
FIG. 9 is a bottom view of the guard and seal assemblies shown in FIG. 8;
FIG. 10 is an exploded view of the seal assembly shown in FIG. 8;
FIG. 11 is a perspective side view of a seal assembly according to yet another embodiment of the present disclosure;
FIG. 12 is a bottom view of the guard and seal assemblies shown in FIG. 11; and
FIG. 13 is an exploded view of the seal assembly shown in FIG. 11.

### DETAILED DESCRIPTION

Particular embodiments of the present disclosure are described hereinbelow with reference to the accompanying drawings; however, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure. Like reference numerals refer to similar or identical elements throughout the description of the figures.

As used herein, the term "distal" refers to that portion of the instrument, or component thereof which is farther from the user while the term "proximal" refers to that portion of the instrument or component thereof which is closer to the user.

Cannulas are employed during, e.g., laparoscopic surgery and may, in various embodiments, provide for the sealed access of laparoscopic surgical instruments into an insufflated body cavity, such as the abdominal cavity. The cannulas of the present disclosure include an instrument valve housing mounted on a cannula tube. Cannulas are typically part of a trocar assembly including a trocar obturator. The trocar obturator is insertable through the cannula to facilitate placement of the cannula through tissue. The cannula and the trocar obturator are separate components but are capable of being selectively connected together. For example, the trocar obturator may be inserted into and through the cannula until the handle of the trocar obturator engages, e.g., selectively locks into, the instrument valve housing of the cannula.

Trocar assemblies are employed to tunnel through an anatomical structure, e.g., the abdominal wall, either by making a new passage through the structure or by passing through an existing opening through the structure. Once the trocar assembly has tunneled through the anatomical structure, the trocar obturator is removed, leaving the cannula in place in the structure, e.g., in the incision created by the trocar assembly. The instrument valve housing of the cannula may include valves that prevent the escape of insufflation gases from the body cavity, while also allowing surgical instruments to be inserted into the cavity.

In various embodiments, a bladeless optical trocar obturator may be provided that permits separation of tissue planes in a surgical procedure and visualization of body tissue fibers as they are being separated, thereby permitting a controlled traversal across a body wall. In other embodiments, the trocar obturator may be bladeless without being optical, e.g., without providing contemporaneous visualization thereof through the distal tip of an obturator. The bladeless obturator may be provided for the blunt dissection of the abdominal lining during a surgical procedure.

Various trocar obturators suitable for use with the cannulas of the present disclosure are known and include, for example, bladed, bladeless, blunt, optical, non-optical. The trocar assemblies will only be described to the extent necessary to disclose the aspects of the present disclosure. For a detailed description of the structure and function of exemplary trocar assemblies, including exemplar trocar obturators and exemplar cannulas, please refer to commonly owned PCT Publication No WO 2016/186905 ("the '905 publication"), the content of which is hereby incorporated by reference herein in its entirety.

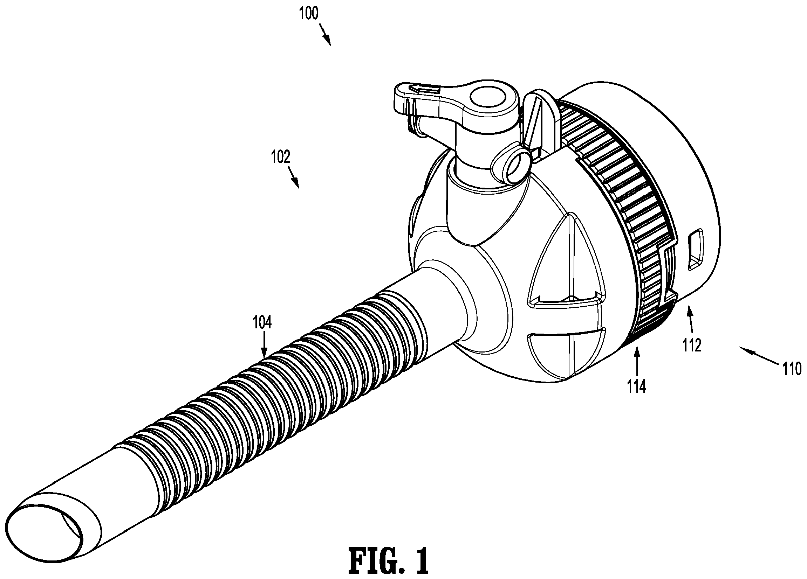

With initial reference now to FIG. 1, a cannula according to aspects of the present disclosure is shown generally as cannula 100. The cannula 100 includes a cannula assembly 102 and an instrument valve housing 110 secured to the cannula assembly 102. The cannula assembly 102 will only be described to the extent necessary to fully disclose the aspects of the present disclosure. For a detailed description of an exemplary cannula assembly, please refer to the '905 publication.

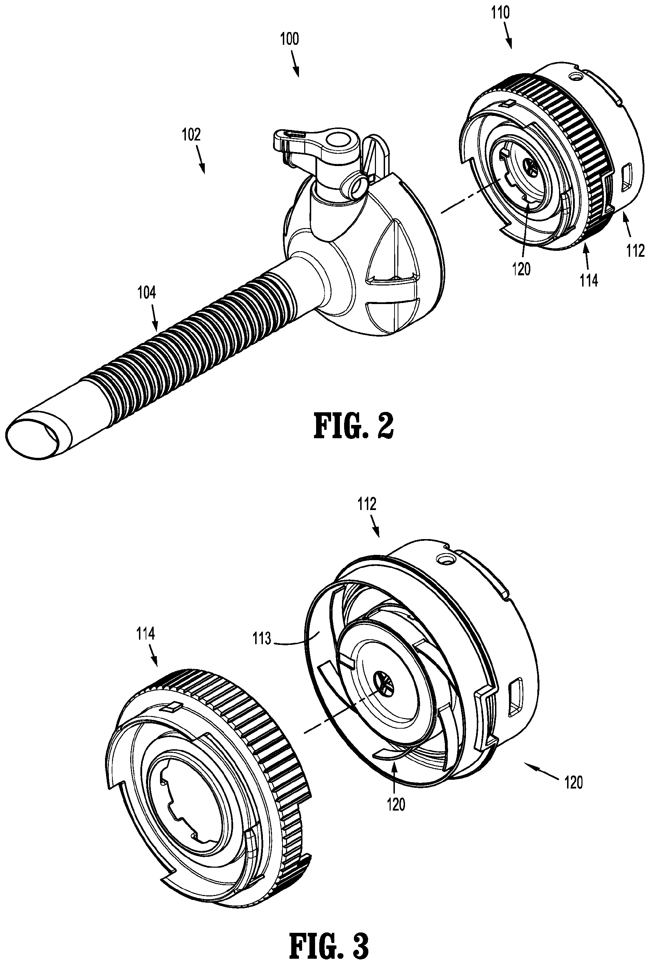

With reference to FIGS. 2 and 3, the instrument valve housing 110 of the cannula 100 includes a first housing section 112 and a second housing section 114. The first and second housing sections 112, 114 are configured to support a valve assembly 120 on a proximal end of the cannula assembly 102. More particularly, at least the second housing section 114 defines a cavity 113 for operably receiving the valve assembly 120. The first housing section 112 of the instrument valve housing 110 may be selectively attachable to, and detachable from, the second housing section 114. The second housing section 114 may be releasably or permanently attached to a cannula tube 104 of the cannula assembly 102. In embodiments, either or both of the first and second housing sections 112, 114 of the instrument valve housing 110 may include knurls, indentations, tabs, or be otherwise configured to facilitate engagement by a clinician.

The first and second housing sections 112, 114 of the instrument valve housing 110 define a longitudinal passage (not shown) for receipt of a surgical instrument (not shown). The valve assembly 120 is supported within the first and second housing sections 112, 114 to provide sealed passage of the surgical instrument through the instrument valve housing 110.

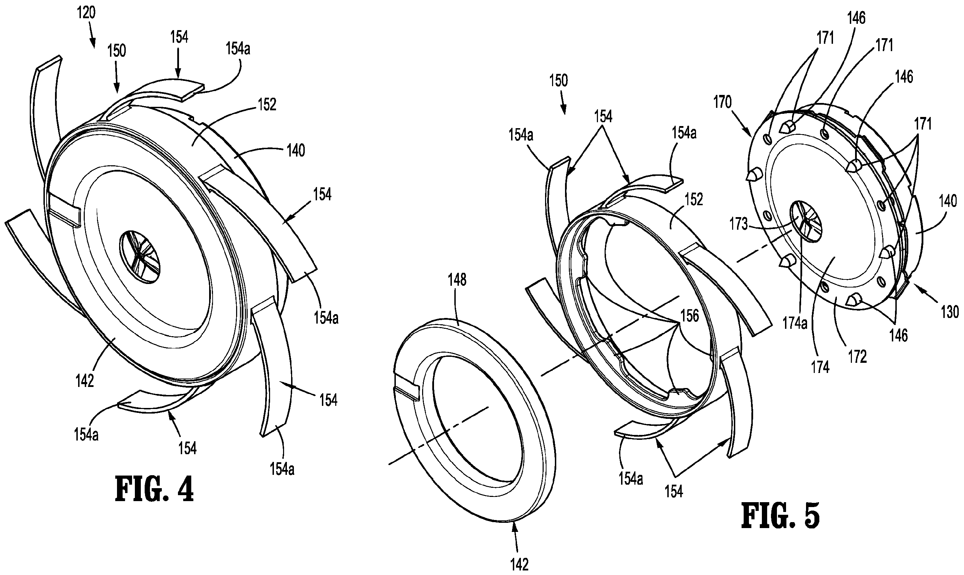

With particular reference to FIGS. 4-7, the valve assembly 120 supported in the instrument valve housing 110 includes a guard assembly 130, a centering mechanism 150, and a seal assembly 170. As will be described in further detail below, the guard assembly 130 is configured to protect the seal assembly 170 during insertion and withdrawal of a surgical instrument through the seal assembly 170. The centering mechanism 150 is configured to permit radial movement of the valve assembly 120 relative to the instrument valve housing 110 when a surgical instrument is received through the valve assembly 120, and return the valve assembly 120 to a centered position once the surgical instrument is withdrawn from within the instrument valve housing 120. The seal assembly 170 provides sealed passage of the surgical instrument through the instrument valve housing 110.

The guard assembly 130 of the valve assembly 120 supported within the instrument valve housing 110 includes first, second, third, and fourth guard members 132, 134, 136, 138. The guard members 132, 134, 136, 138 are formed of elastomeric materials or other suitable material. Each of the first, second, third, and fourth guard members 132, 134, 136, 138 includes a base portion 132a, 134a, 136a, 138a, and a flap portion 132b, 134b, 136b, 138b extending from the respective base portions 132a, 134a, 136a, 138a. The base portions 132a, 134a, 136a, 138a of the first, second, third, and fourth guard members 132, 134, 136, 138 each define a plurality of openings 133, 135, 137, 139 respectively. Each opening of the plurality of openings 131, 133, 135, 137 is configured to receive a pin of a plurality of pins 146 of an upper retainer member 140. When the guard assembly 130 is assembled, the base portions 132a, 134a, 136a, 138a of the respective first, second, third, and fourth guard members 132, 134, 136, 138 define an annular member with the respective flap portions 132b, 134b, 136b, 138b extending radially inward.

Each of the flap portions 132b, 134b, 136b, 138b of the respective first, second, third, and fourth guard members 132, 134, 136, 138 includes first and second flap sections 133a, 133b, 135a, 135b, 137a, 137b, 139a, 139b, respectively, separated by a radial channel or groove 133c, 135c, 137c, 139c. Each of the first and second flap sections 133a, 133b, 135a, 135b, 137a, 137b, 139a, 139b of the respective flap portions 132b, 134b, 136b, 138b tapers radially inward and downward. The first and second flap sections 133a, 133b, 135a, 135b, 137a, 137b, 139a, 139b and the radial channels 133c, 135c, 137c, 139c operate as instrument guides directing a surgical instrument through the flap portions 132b, 134b, 136b, 138b. As shown, the flap portions 132b, 134b, 136b, 138b of the respective first, second, third, and fourth guard members 132, 134, 136, 138 do not overlap, although it is envisioned that the guard members 132, 134, 136, 138 may be modified such that at least a portion of each of the first, second, third, and fourth guard member 132, 134, 136, 138 overlaps one or both adjacent guard members.

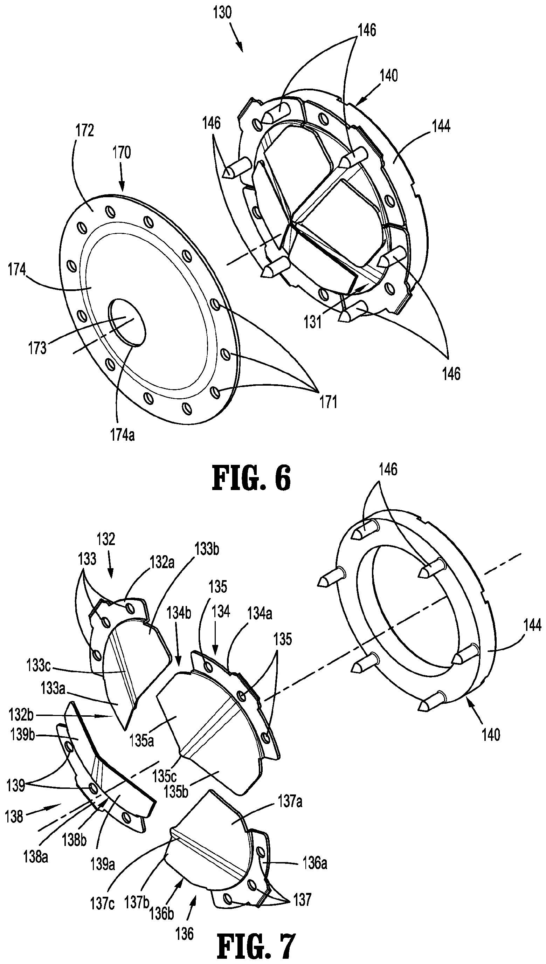

As shown in FIGS. 6 and 7, the first, second, third, and fourth guard members 132, 134, 136, 138 are arranged adjacent one another with the base portions 132a, 134a, 136a, 138a forming the annular member, and the flap portions 132b, 134b, 136b, 138b occupying the opening 131 (FIG. 6) defined by the annular member, and thus, substantially covering the seal assembly 170.

The flap portions 132b, 134b, 136b, 138b of the respective first, second, third and fourth guard members 132, 134, 136, 138 are configured to flex downward upon engagement with a surgical instrument to facilitate passage of the surgical instrument through the seal assembly 170. More particularly, engagement of one or more of the flap portions 132b, 134b, 136b, 138b of the first, second, third, and fourth guard members 132, 134, 136, 138, respectively, flexes one or more of the flap portions 132b, 134b, 136b, 138b downward into engagement with a septum seal 174 of the seal assembly 170 to stretch the septum seal 174 of the seal assembly 170 to increase the size of a central opening 173 of the septum seal 174. The increased size of the central opening 173 of the septum seal 174 permits receipt of the surgical instrument through the valve assembly 120. The larger the diameter of the surgical instrument, the more the first, second, third, and fourth flap portions 132b, 134b, 136b, 138b of the first, second, third, and fourth guard members 132, 134, 136, 138 are flexed downward and the greater the size of the central opening 173 in the septum seal 174. The flap portions 132b, 134b, 136b, 138b also operate to guide and orient the surgical instrument through the seal assembly 170. Additionally, the flap portions 132b, 134b, 136b, 138b may also inhibit inversion of the seal assembly 170 during withdrawal of a surgical instrument from the seal assembly 170.

It is envisioned that the guard assembly 130 may include a guard member (not shown) including two pairs of opposed flap portions (not shown) supported on a single base portion. The guard assembly may include any number of guard members, and the guard members may include flap portions of any size or configuration.

With continued reference to FIG. 5-7, the guard assembly 130 further includes upper and lower retainer members 140, 142 for retaining the first, second, third and fourth guard members 132, 134, 136, 138 relative to one another. More particularly, the upper retainer member 140 includes a base 144 and the plurality of fingers or pins 146 extending downwardly from the base 144. The lower retainer member 142 includes a base 148 defining an annular channel (not shown) for securely receiving the plurality of pins 146 of the upper retainer member 140.

Although the plurality of pins 146 is shown as extending downwardly from the base 144 of the upper retainer member 140 for engagement with the lower retainer member 142, in other embodiments, the plurality of pins 146 may instead extend upwardly from the base 148 of the lower retainer member 142 for engagement with the upper retainer member 140, or the pins 146 may extend from both the upper and lower retainer members 140, 142 and extend both upwardly and downwardly. In addition, while the lower retainer member 142 is described as including an annular channel, the lower retainer member 142 may instead include one or more discrete openings (not shown) for receiving the corresponding fingers or pins 146, which may improve the engagement of the pins 146 with the lower retainer member 142 and increase the retention between the pins 146 and the lower retainer member 142 once the upper and lower retainer members 140, 142 are connected to each other. Employing an annular channel instead of the discrete openings eliminates the need to circumferential align the upper and lower retainer members 140, 142 prior to connecting the upper and lower retainer members 140, 142 to one another.

The plurality of pins 146 of the upper retainer member 140 of the guard assembly 130 are configured to be received through the plurality of openings 131, 133, 135, 137 in the respective base portions 132a, 134a, 136a, 138a of the first, second, third, and fourth guard members 132, 134, 136, 138, respectively. Receipt of the plurality of pins 146 of the upper retainer member 140 of the guard assembly 130 through the plurality of openings 133, 135, 137, 139 in the respective first, second, third, and fourth guard members 132, 134, 136, 138 and subsequent attachment of the lower retainer member 142 to the upper retainer member 140 secures the first, second, third, and fourth guard members 132, 134, 136, 138 relative to one another.

With particular reference to FIGS. 4 and 5, the centering mechanism 150 of the instrument valve housing 110 is configured to maintain the valve assembly 110 centered within the cavity 113 (FIG. 3) of the first and second housing sections 112, 114 of the instrument valve housing 110. More particularly, the centering mechanism 150 includes an annular ring 152 and a plurality of spokes or spring elements 154 extending radially outward from the annular ring 152. The centering mechanism 150 further includes a plurality of tabs 156 that extend inwardly from the annular ring 152 and are configured to engage the seal member 170. For a detailed description of the structure and function of an exemplary centering mechanism, please refer to commonly owned U.S. Pat. App. Pub. No. 2015/0025477 ("the '477 publication"), the content of which is incorporated herein by reference in its entirety. It is envisioned that the centering mechanism 150 may include two centering mechanisms, as disclosed in the '477 publication.

As described in the '477 publication, each spoke of the plurality of spokes 154 of the centering mechanism 150 each include a free end 154a configured to engage an inner wall 114a (FIG. 3) of the upper housing section 112 when the valve assembly 120 is moved off-center to bias the valve assembly 120 back to a centered position.

Alternatively, the centering mechanism may include a bellows or otherwise be configured to maintain the valve assembly 120 centered within the instrument valve housing 110.

With additional reference to FIG. 6, the seal member 170 of the valve assembly 120 is configured to provide a seal around an outer surface of a surgical instrument passing through the instrument valve housing 110. The seal member 170 includes an annular base portion 172, and the septum seal 174 is supported by the annular base portion 172. In embodiments, the septum seal 174 may be integrally formed with the annular base portion 172. Alternatively, the septum seal 174 is secured to the annular base portion 172 with adhesive, welding, friction fit, or in any other suitable manner.

The annular base portion 172 of the seal assembly 170 defines a plurality of openings 171 corresponding to the pins 146 extending from the upper retainer member 140 of the guard assembly 130. Receipt of the pins 146 through the openings 171 in the annular base portion 172 of the seal assembly 170 secures the seal assembly 170 relative to the guard assembly 130.

The septum seal 174 of the seal member 170 is formed of an elastic material, e.g., rubber, and defines a central opening 173. In embodiments, the septum seal 174 may include one or more fabric layers. The septum seal 174 is configured to provide a seal around an outer surface of a surgical instrument passing through the valve assembly 120. A surface 174a of the septum seal 174 defining the central opening 173 may be beveled or otherwise configured to facilitate reception of the surgical instrument through the central opening 173 in the septum seal 174.

With particular reference to FIGS. 4 and 5, the upper retainer member 140 of the guard assembly 130 of the valve assembly 120 is positioned relative to the seal member 170 such that the pins 146 of the upper retainer member 140 are received through the openings 171 (FIG. 6) in the annular body portion 172 of the seal member 170. Prior to receiving the pins 146 of the upper retainer member 140 through the openings 171 in the annular body portion 172, the first, second, third and fourth guard members 132, 134, 136, 138 of the guard assembly 130 are secured to the upper retainer member 140. In this manner, when the pins 146 of the upper retainer member 140 are received through the openings 171 in the annular body portion 172, the first, second, third, and fourth guard members 132, 134, 136, 138 of the guard assembly 130 are positioned adjacent to and covering the septum seal 174.

The combined upper retainer member 140, the first, second, third and fourth guard members 132, 134, 136, 138, and the seal assembly 170 are then received within the annular ring 152 of the centering mechanism 150. The lower retainer member 142 of the guard assembly 130 is then secured to the upper retainer member 140. Receipt of the tabs 156 of the centering mechanism 150 the upper retainer member 140 and the lower retainer member secures the guard assembly 130 and the seal assembly 170 within the annular ring 152 of the centering mechanism 150.

During a surgical procedure utilizing cannula 100, an instrument (not shown) is introduced into the instrument valve housing 100 through the longitudinal passage in the first and second housing sections 112, 114. As described above, the distal end of the instrument engages one or more of the flap portions 132b, 134b, 136b, 138b of the respective first, second, third, and fourth guard members 132, 134, 136, 138. The one or more first, second, third, and fourth flap portions 132b, 134b, 136b, 138b flex downward into contact with the septum seal 174 of the seal member 170 to cause the central opening 173 of the septum seal 174 to open to accommodate passage of the surgical instrument through the septum seal 174.

The first, second, third, and fourth guard members 132, 134, 136, 138 of the guard assembly 130 operate to protect the septum seal 174 of the seal assembly 170 from tearing or other damage as a surgical instrument is received through the seal assembly 170. Additionally, the guard assembly 130 may prevent inversion of the septum seal 174 as the surgical instrument is retracted through the septum seal 174.

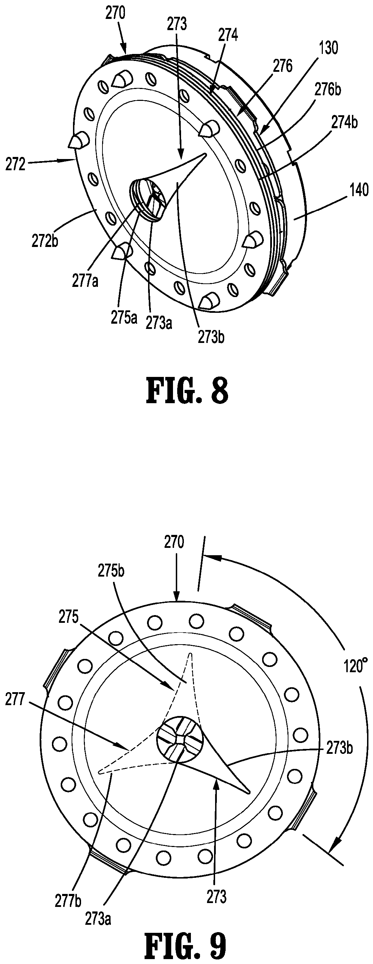

With reference now to FIGS. 8-10, a seal assembly according to another embodiment of the present disclosure is shown generally as seal assembly 270. The seal assembly 270 includes first, second, and third seal members 272, 274, 276. Each of the first, second, and third seal members 272, 274, 276 includes an annular base portion 272a, 274a, 276a and a septum seal 272b, 274b, 276b supported by the respective annular base portion 272a, 274a, 276a. Each of the first, second, and third seal members 272, 274, 276 are substantially similar to the seal assembly 170 described hereinabove, and will only be described in detail as relates to the differences therebetween.

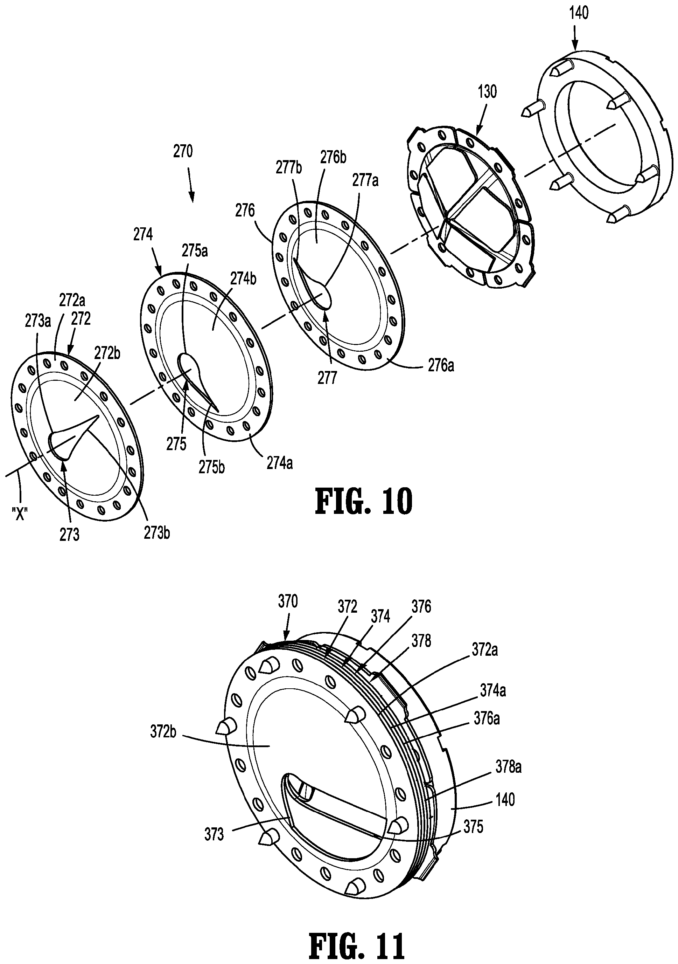

Each septum seal 272b, 274b, 276b of the respective first, second, and third seal members 272, 274, 276 defines opening 273, 275, 277 having a substantially teardrop shape. More particularly, each of the openings 273, 275, 277 includes a circular portion 273a, 275a, 277a in axial alignment with a longitudinal axis "x" (FIG. 10) of the seal assembly 270 and a triangular portion 273b, 275b, 277b in communication with the circular portion 273a, 275a, 277a and extending radially outward toward the respective annular base portion 272a, 274a, 276a of the first, second, and third seal members 272, 274, 276, respectively.

The first, second and third seal members 272, 274, 276 of the seal assembly 270 are configured to receive a surgical instrument in a sealed manner. More particularly, the first, second and third seal members 272, 274, 276 are stacked on top of each other and are indexed one-hundred twenty degrees (120°) from one another. In this manner, the circular portions 273a, 275a, 277a of the respective openings 273, 275, 277 are aligned with each other and the triangular portions 273b, 275b, 277b of each of the seal members 272, 274, 276 are spaced one-hundred twenty degrees (120°) from the triangular portions 273b, 275b, 277b of the adjacent seal members 272, 274, 276.

The seal assembly 270 is supported by the upper and retainer members 140, 142 (FIG. 5). The seal assembly 270 may be used in combination with the guard assembly 130 to prevent tearing of the septum seals 272b, 274b, 276b of the respective first, second, and third seal members 272, 274, 276 as a surgical instrument is received through the seal assembly 270. The configuration of the openings 273, 275, 277 in the septum seals 272b, 274b, 276b, respectively, of the respective first, second and third seal members 272, 274, 276 provides improved sealing about a surgical instrument as the surgical instrument is inserted through and withdrawn from the seal assembly 270 and during manipulation of the surgical instrument.

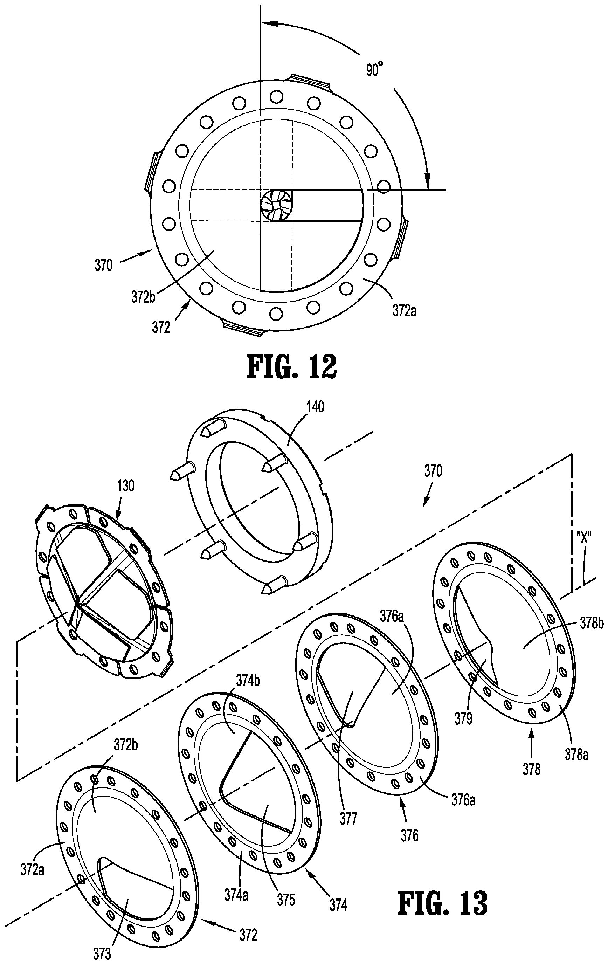

With reference now to FIGS. 11-13, a seal assembly according to another embodiment of the present disclosure is shown generally as seal assembly 370. The seal assembly 370 includes first, second, third and fourth seal members 372, 374, 376, 378. Each of the first, second, third, and fourth seal members 372, 374, 376, 378 includes an annular base portion 372a, 374a, 376a, 378a and a septum seal 372b, 374b, 376b, 378b supported by the respective annular base portion 372a, 374a, 376a, 378a. Each of the first, second, third and fourth seal members 372, 374, 376, 378 are substantially similar to the first, second, and third seal members 272, 274, 276 of the seal assembly 270 described hereinabove, and will only be described in detail as relates to the differences therebetween.

Each septum seal 372b, 374b, 376b, 378b of the respective first, second, third and fourth seal members 372, 374, 376, 378 defines opening 373, 375, 377, 379 including a sector of the respective septum seals 372b, 374b, 376b, 378b. More particularly, and as shown, each of the openings 373, 375, 377, 379 occupies one quarter (1/4) of the area of the respective septum seals 372b, 374b, 376b, 378b. It is envisioned that the openings 373, 375, 377, 379 may occupy more or less than a quarter of the respective septum seals 372b, 374b, 376b, 378b.

The first, second, third and fourth seal members 372, 374, 376, 378 of the seal assembly 370 are configured to receive a surgical instrument in a sealed manner. More particularly, the first, second, third and fourth seal members 372, 374, 376, 378 are stacked on top of each other and are indexed ninety degrees (90°) from one another. In this manner, the septum seal 372b, 374b, 376b, 378b of the respective first, second, third and fourth seal members 372, 374, 376, 378 covers the openings 373, 375, 377, 379 in the adjacent opening septum seals 372b, 374b, 376b, 378b.

The seal assembly 370 is supported by the upper and retainer members 140, 142 (FIG. 5). The guard assembly 130 may be used in combination with the seal assembly 370 to prevent tearing of the septum seals 372b, 374b, 376b, 378b of the respective first, second, third and fourth seal members 372, 374, 376, 378 as a surgical instrument is received through the seal assembly 370.

The configuration of the openings 373, 375, 377, 379 in the septum seals 372b, 374b, 376b, 378b, respectively, of the respective first, second, third and fourth seal members 372, 374, 376, 378 provides improved sealing about a surgical instrument as the surgical instrument is inserted through and withdrawn from the seal assembly 370 and during manipulation of the surgical instrument.

While various embodiments of the present disclosure have been shown and described herein, it will be obvious to those skilled in the art that these embodiments are provided by way of example only. Numerous variations, changes, and substitutions will now occur to those skilled in the art without departing from the present disclosure. Accordingly, it is intended that the invention be limited only by the spirit and scope of the appended claims.

The invention may be described by reference to the following numbered paragraphs:
1. A cannula comprising: an instrument valve housing including first and second housing sections and defining a cavity; and a valve assembly disposed within the cavity of the instrument valve housing, the valve assembly including: a seal assembly including at least one seal member, the at least one seal member including a ring portion and a septum seal extending across the ring portion, a guard assembly supporting the seal assembly, the guard assembly including a plurality of guard members, each guard member of the plurality of guard members having a ring portion and a flap portion, the flap portion of each guard member of the plurality of guard members including first and second flap sections and a groove disposed between the first and second flap sections, a centering mechanism including an annular ring and a plurality of spokes extending outwardly from the annular ring, the seal and guard assemblies being supported within the annular ring and the annular ring being disposed within the cavity of the instrument valve housing, wherein the flap portions of the plurality of guard members of the guard assembly are configured to engage and stretch the septum seal during reception of a surgical instrument through the valve assembly.
2. The cannula of paragraph 1, wherein the guard assembly includes first, second, third and fourth guard members.
3. The cannula of paragraph 2, wherein the ring portions of the first, second, third and fourth guard members define an annular member.
4. The cannula of paragraph 2, wherein the first and second flap sections of the first, second, third and fourth guard members are tapered radially inward.
5. The cannula of paragraph 4, wherein the groove in each of the first, second, third and fourth guard members extends radially inward.
6. The cannula of paragraph 1, wherein the at least one seal member includes three seal members.
7. The cannula of paragraph 6, wherein each of the three seal members includes a septum seal defining an opening having a substantial tear-drop shape.
8. The cannula of paragraph 7, wherein each seal member of the three seal members is disposed relative to the other seal members such that the openings in the septum seals are one-hundred twenty degrees apart.
9. The cannula of paragraph 1, wherein the seal assembly includes four seal members.
10. The cannula of paragraph 9, wherein each of the four seal members includes a septum seal defining a sector of the septum seal.
11. The cannula of paragraph 10, wherein the sector includes one quarter of the septum seal.
12. The cannula of paragraph 11, wherein each seal member of the four seal members is disposed relative to the other seal members such that the openings in the septum seals are oriented ninety degrees apart.

## Claims

1. A cannula comprising:
an instrument valve housing including first and second housing sections and defining a cavity; and
a valve assembly disposed within the cavity of the instrument valve housing, the valve assembly including:
a seal assembly including at least one seal member, the at least one seal member including a ring portion and a septum seal extending across the ring portion,
a guard assembly supporting the seal assembly, the guard assembly including a plurality of guard members, each guard member of the plurality of guard members having a ring portion and a flap portion, the flap portion of each guard member of the plurality of guard members including first and second flap sections and a groove disposed between the first and second flap sections,
a centering mechanism including an annular ring and a plurality of spokes extending outwardly from the annular ring, the seal and guard assemblies being supported within the annular ring and the annular ring being disposed within the cavity of the instrument valve housing, wherein the flap portions of the plurality of guard members of the guard assembly are configured to engage and stretch the septum seal during reception of a surgical instrument through the valve assembly.

2. The cannula of claim 1, wherein the guard assembly includes first, second, third and fourth guard members.

3. The cannula of claim 2, wherein the ring portions of the first, second, third and fourth guard members define an annular member.

4. The cannula of claim 2, wherein the first and second flap sections of the first, second, third and fourth guard members are tapered radially inward.

5. The cannula of claim 4, wherein the groove in each of the first, second, third and fourth guard members extends radially inward.

6. The cannula of any preceding claim, wherein the at least one seal member includes three seal members.

7. The cannula of claim 6, wherein each of the three seal members includes a septum seal defining an opening having a substantial tear-drop shape.

8. The cannula of claim 7, wherein each seal member of the three seal members is disposed relative to the other seal members such that the openings in the septum seals are one-hundred twenty degrees apart.

9. The cannula of any preceding claim, wherein the seal assembly includes four seal members.

10. The cannula of claim 9, wherein each of the four seal members includes a septum seal defining a sector of the septum seal.

11. The cannula of claim 10, wherein the sector includes one quarter of the septum seal.

12. The cannula of claim 11, wherein each seal member of the four seal members is disposed relative to the other seal members such that the openings in the septum seals are oriented ninety degrees apart.
